# EUROPEAN PATENT APPLICATION

(11) **EP 1 288 312 A2**
(43) Date of publication of application: **05.03.2003**
(21) Application number: 02255875.3
(22) Date of filing: 22.08.2002
(51) Int. Cl.: C12Q 1/68

(54) **Method for determining quinolone resistance of tubercle bacilli**

(30) Priority: 04.09.2001 JP 2001267769
(71) Applicant: Osaka Prefectural Institute of Republic Health, Osaka-shi, Osaka (JP); NISSHINBO INDUSTRIES, INC., Chuo-ku, Tokyo (JP)
(72) Inventor: Suzuki, Yasuhiko, c/o Osaka Prefectural Institute, Higashinari-ku, Osaka-shi, Osaka (JP); Ichihara, Tatsuo, c/o Nisshinbo Industries, Inc., Chiba-shi, Chiba (JP); Satoh, Noriko, c/o Nisshinbo Industries, Inc., Chiba-shi, Chiba (JP)
(74) Representative: Tombling, Adrian George

(57) **Abstract**

A novel mutation in the gyrase A gene involved in quinolon resistance phenotype acquisition of tubercle bacillus is identified and utilized to provide a method and kit for determining quinolon resistance of tubercle bacillus.

SEQUENCE LISTING

<110> Nisshinbo Industries, Inc.
      Osakafu

<120> Method for Determining Quinolon Resistance of Tubercle bacilli

<130> OP1354

<150> JP 2001-267769
<151> 2001-09-04

<160> 6

<170> PatentIn Ver. 2.0

<210> 1
<211> 50
<212> DNA
<213> Mycobacterium tuberculosis

<220>
<221> CDS
<222> (2)..(49)

<400> 1
c aac tac cac ccg cac ggc gac gcg tcg atc tac gac agc ctg gtg cgc 49
  Asn Tyr His Pro His Gly Asp Ala Ser Ile Tyr Asp Ser Leu Val Arg
    1               5                  10                  15
a                                                                 50

<210> 2
<211> 16
<212> PRT
<213> Mycobacterium tuberculosis

<400> 2
Asn Tyr His Pro His Gly Asp Ala Ser Ile Tyr Asp Ser Leu Val Arg

1               5                   10                  15

<210> 3
<211> 2884
<212> DNA
<213> Mycobacterium tuberculosis

<220>
<221> CDS
<222> (266)..(2782)

<400> 3
aagatcaaca aggaagacgg cattcagcgg tacaagggtc taggtgaaat ggacgctaag 60
gagttgtggg agaccaccat ggatccctcg gttcgtgtgt tgcgtcaagt gacgctggac 120
gacgccgccg ccgccgacga gttgttctcc atcctgatgg gcgaggacgt cgacgcgcgg 180
cgcagcttta tcacccgcaa cgccaaggat gttcggttcc tggatgtcta acgcaaccct 240
gcgttcgatt gcaaacgagg aatag atg aca gac acg acg ttg ccg cct gac   292
                            Met Thr Asp Thr Thr Leu Pro Pro Asp
                            1               5
gac tcg ctc gac cgg atc gaa ccg gtt gac atc gag cag gag atg cag   340
Asp Ser Leu Asp Arg Ile Glu Pro Val Asp Ile Glu Gln Glu Met Gln
10                  15                  20                  25
cgc agc tac atc gac tat gcg atg agc gtg atc gtc ggc cgc gcg ctg   388
Arg Ser Tyr Ile Asp Tyr Ala Met Ser Val Ile Val Gly Arg Ala Leu
                30                  35                  40
ccg gag gtg cgc gac ggg ctc aag ccc gtg cat cgc cgg gtg ctc tat   436
Pro Glu Val Arg Asp Gly Leu Lys Pro Val His Arg Arg Val Leu Tyr
            45                  50                  55
gca atg ttc gat tcc ggc ttc cgc ccg gac cgc agc cac gcc aag tcg   484
Ala Met Phe Asp Ser Gly Phe Arg Pro Asp Arg Ser His Ala Lys Ser
        60                  65                  70
gcc cgg tcg gtt gcc gag acc atg ggc aac tac cac ccg cac ggc gac   532
Ala Arg Ser Val Ala Glu Thr Met Gly Asn Tyr His Pro His Gly Asp
    75                  80                  85
gcg tcg atc tac gac agc ctg gtg cgc atg gcc cag ccc tgg tcg ctg   580
Ala Ser Ile Tyr Asp Ser Leu Val Arg Met Ala Gln Pro Trp Ser Leu
90                  95                  100                 105
cgc tac ccg ctg gtg gac ggc cag ggc aac ttc ggc tcg cca ggc aat   628
Arg Tyr Pro Leu Val Asp Gly Gln Gly Asn Phe Gly Ser Pro Gly Asn
                110                 115                 120
gac cca ccg gcg gcg atg agg tac acc gaa gcc cgg ctg acc ccg ttg   676

Asp Pro Pro Ala Ala Met Arg Tyr Thr Glu Ala Arg Leu Thr Pro Leu
            125                 130                 135
gcg atg gag atg ctg agg gaa atc gac gag gag aca gtc gat ttc atc 724
Ala Met Glu Met Leu Arg Glu Ile Asp Glu Glu Thr Val Asp Phe Ile
        140                 145                 150
cct aac tac gac ggc cgg gtg caa gag ccg acg gtg cta ccc agc cgg 772
Pro Asn Tyr Asp Gly Arg Val Gln Glu Pro Thr Val Leu Pro Ser Arg
    155                 160                 165
ttc ccc aac ctg ctg gcc aac ggg tca ggc ggc atc gcg gtc ggc atg 820
Phe Pro Asn Leu Leu Ala Asn Gly Ser Gly Gly Ile Ala Val Gly Met
170                 175                 180                 185
gca acc aat atc ccg ccg cac aac ctg cgt gag ctg gcc gac gcg gtg 868
Ala Thr Asn Ile Pro Pro His Asn Leu Arg Glu Leu Ala Asp Ala Val
                190                 195                 200
ttc tgg gcg ctg gag aat cac gac gcc gac gaa gag gag acc ctg gcc 916
Phe Trp Ala Leu Glu Asn His Asp Ala Asp Glu Glu Glu Thr Leu Ala
            205                 210                 215
gcg gtc atg ggg cgg gtt aaa ggc ccg gac ttc ccg acc gcc gga ctg 964
Ala Val Met Gly Arg Val Lys Gly Pro Asp Phe Pro Thr Ala Gly Leu
        220                 225                 230
atc gtc gga tcc cag ggc acc gct gat gcc tac aaa act ggc cgc ggc 1012
Ile Val Gly Ser Gln Gly Thr Ala Asp Ala Tyr Lys Thr Gly Arg Gly
    235                 240                 245
tcc att cga atg cgc gga gtt gtt gag gta gaa gag gat tcc cgc ggt 1060
Ser Ile Arg Met Arg Gly Val Val Glu Val Glu Glu Asp Ser Arg Gly
250                 255                 260                 265
cgt acc tcg ctg gtg atc acc gag ttg ccg tat cag gtc aac cac gac 1108
Arg Thr Ser Leu Val Ile Thr Glu Leu Pro Tyr Gln Val Asn His Asp
                270                 275                 280
aac ttc atc act tcg atc gcc gaa cag gtc cga gac ggc aag ctg gcc 1156
Asn Phe Ile Thr Ser Ile Ala Glu Gln Val Arg Asp Gly Lys Leu Ala
            285                 290                 295
ggc att tcc aac att gag gac cag tct agc gat cgg gtc ggt tta cgc 1204
Gly Ile Ser Asn Ile Glu Asp Gln Ser Ser Asp Arg Val Gly Leu Arg
        300                 305                 310
atc gtc atc gag atc aag cgc gat gcg gtg gcc aag gtg gtg atc aat 1252
Ile Val Ile Glu Ile Lys Arg Asp Ala Val Ala Lys Val Val Ile Asn
    315                 320                 325
aac ctt tac aag cac acc cag ctg cag acc agc ttt ggc gcc aac atg 1300
Asn Leu Tyr Lys His Thr Gln Leu Gln Thr Ser Phe Gly Ala Asn Met
330                 335                 340                 345

cta gcg atc gtc gac ggg gtg ccg cgc acg ctg cga ctg gac cag ctg 1348
Leu Ala Ile Val Asp Gly Val Pro Arg Thr Leu Arg Leu Asp Gln Leu
                350                 355                 360
atc cgc tat tac gtt gac cac caa ctc gac gtc att gtg cgg cgc acc 1396
Ile Arg Tyr Tyr Val Asp His Gln Leu Asp Val Ile Val Arg Arg Thr
            365                 370                 375
acc tac cgg ctg cgc aag gca aac gag cga gcc cac att ctg cgc ggc 1444
Thr Tyr Arg Leu Arg Lys Ala Asn Glu Arg Ala His Ile Leu Arg Gly
        380                 385                 390
ctg gtt aaa gcg ctc gac gcg ctg gac gag gtc att gca ctg atc cgg 1492
Leu Val Lys Ala Leu Asp Ala Leu Asp Glu Val Ile Ala Leu Ile Arg
    395                 400                 405
gcg tcg gag acc gtc gat atc gcc cgg gcc gga ctg atc gag ctg ctc 1540
Ala Ser Glu Thr Val Asp Ile Ala Arg Ala Gly Leu Ile Glu Leu Leu
410                 415                 420                 425
gac atc gac gag atc cag gcc cag gca atc ctg gac atg cag ttg cgg 1588
Asp Ile Asp Glu Ile Gln Ala Gln Ala Ile Leu Asp Met Gln Leu Arg
                430                 435                 440
cgc ctg gcc gca ctg gaa cgc cag cgc atc atc gac gac ctg gcc aaa 1636
Arg Leu Ala Ala Leu Glu Arg Gln Arg Ile Ile Asp Asp Leu Ala Lys
            445                 450                 455
atc gag gcc gag atc gcc gat ctg gaa gac atc ctg gca aaa ccc gag 1684
Ile Glu Ala Glu Ile Ala Asp Leu Glu Asp Ile Leu Ala Lys Pro Glu
        460                 465                 470
cgg cag cgt ggg atc gtg cgc gac gaa ctc gcc gaa atc gtg gac agg 1732
Arg Gln Arg Gly Ile Val Arg Asp Glu Leu Ala Glu Ile Val Asp Arg
    475                 480                 485
cac ggc gac gac cgg cgt acc cgg atc atc gcg gcc gac gga gac gtc 1780
His Gly Asp Asp Arg Arg Thr Arg Ile Ile Ala Ala Asp Gly Asp Val
490                 495                 500                 505
agc gac gag gat ttg atc gcc cgc gag gac gtc gtt gtc act atc acc 1828
Ser Asp Glu Asp Leu Ile Ala Arg Glu Asp Val Val Val Thr Ile Thr
                510                 515                 520
gaa acg gga tac gcc aag cgc acc aag acc gat ctg tat cgc agc cag 1876
Glu Thr Gly Tyr Ala Lys Arg Thr Lys Thr Asp Leu Tyr Arg Ser Gln
            525                 530                 535
aaa cgc ggc ggc aag ggc gtg cag ggt gcg ggg ttg aag cag gac gac 1924
Lys Arg Gly Gly Lys Gly Val Gln Gly Ala Gly Leu Lys Gln Asp Asp
        540                 545                 550
atc gtc gcg cac ttc ttc gtg tgc tcc acc cac gat ttg atc ctg ttc 1972
Ile Val Ala His Phe Phe Val Cys Ser Thr His Asp Leu Ile Leu Phe

555                 560                 565
ttc acc acc cag gga cgg gtt tat cgg gcc aag gcc tac gac ttg ccc    2020
Phe Thr Thr Gln Gly Arg Val Tyr Arg Ala Lys Ala Tyr Asp Leu Pro
570                 575                 580                 585
gag gcc tcc cgg acg gcg cgc ggg cag cac gtg gcc aac ctg tta gcc    2068
Glu Ala Ser Arg Thr Ala Arg Gly Gln His Val Ala Asn Leu Leu Ala
                590                 595                 600
ttc cag ccc gag gaa cgc atc gcc cag gtc atc cag att cgc ggc tac    2116
Phe Gln Pro Glu Glu Arg Ile Ala Gln Val Ile Gln Ile Arg Gly Tyr
            605                 610                 615
acc gac gcc ccg tac ctg gtg ctg gcc act cgc aac ggg ctg gtg aaa    2164
Thr Asp Ala Pro Tyr Leu Val Leu Ala Thr Arg Asn Gly Leu Val Lys
        620                 625                 630
aag tcc aag ctg acc gac ttc gac tcc aat cgc tcg ggc gga atc gtg    2212
Lys Ser Lys Leu Thr Asp Phe Asp Ser Asn Arg Ser Gly Gly Ile Val
    635                 640                 645
gcg gtc aac ctg cgc gac aac gac gag ctg gtc ggt gcg gtg ctg tgt    2260
Ala Val Asn Leu Arg Asp Asn Asp Glu Leu Val Gly Ala Val Leu Cys
650                 655                 660                 665
tcg gcc ggc gac gac ctg ctg ctg gtc tcg gcc aac ggg cag tcc atc    2308
Ser Ala Gly Asp Asp Leu Leu Leu Val Ser Ala Asn Gly Gln Ser Ile
                670                 675                 680
agg ttc tcg gcg acc gac gag gcg ctg cgg cca atg ggt cgt gcc acc    2356
Arg Phe Ser Ala Thr Asp Glu Ala Leu Arg Pro Met Gly Arg Ala Thr
            685                 690                 695
tcg ggt gtg cag ggc atg cgg ttc aat atc gac gac cgg ctc gtg tcg    2404
Ser Gly Val Gln Gly Met Arg Phe Asn Ile Asp Asp Arg Leu Val Ser
        700                 705                 710
ctg aac gtc gtg cgt gaa ggc acc tat ctg ctg gtg gcg acg tca ggg    2452
Leu Asn Val Val Arg Glu Gly Thr Tyr Leu Leu Val Ala Thr Ser Gly
    715                 720                 725
ggc tat gcg aaa cgt acc gcg atc gag gaa tac ccg gta cag ggc cgc    2500
Gly Tyr Ala Lys Arg Thr Ala Ile Glu Glu Tyr Pro Val Gln Gly Arg
730                 735                 740                 745
ggc ggt aaa ggt gtg ctg acg gtc atg tac gac cgc cgg cgc ggc agg    2548
Gly Gly Lys Gly Val Leu Thr Val Met Tyr Asp Arg Arg Arg Gly Arg
                750                 755                 760
ttg gtt ggg gcg ttg att gtc gac gac gac agc gag ctg tat gcc gtc    2596
Leu Val Gly Ala Leu Ile Val Asp Asp Asp Ser Glu Leu Tyr Ala Val
            765                 770                 775
act tcc ggc ggt ggc gtg atc cgc acc gcg gca cgc cag gtt cgc aag    2644

Thr Ser Gly Gly Gly Val Ile Arg Thr Ala Ala Arg Gln Val Arg Lys
        780                 785                 790
gcg gga cgg cag acc aag ggt gtt cgg ttg atg aat ctg ggc gag ggc    2692
Ala Gly Arg Gln Thr Lys Gly Val Arg Leu Met Asn Leu Gly Glu Gly
    795                 800                 805
gac aca ctg ttg gcc atc gcg cgc aac gcc gaa gaa agt ggc gac gat    2740
Asp Thr Leu Leu Ala Ile Ala Arg Asn Ala Glu Glu Ser Gly Asp Asp
810                 815                 820                 825
aat gcc gtg gac gcc aac ggc gca gac cag acg ggc aat taa            2782
Asn Ala Val Asp Ala Asn Gly Ala Asp Gln Thr Gly Asn
                830                 835
tcaggctcgc ccgacgacga tgcggatcgc gtagcgatct gaggaggaat cgggcagcta 2842
ggctcggcag ccgggtacga gtgttaggag tcggggtgac tg                    2884

<210> 4
<211> 838
<212> PRT
<213> Mycobacterium tuberculosis

<400> 4
Met Thr Asp Thr Thr Leu Pro Pro Asp Asp Ser Leu Asp Arg Ile Glu
 1               5                  10                  15
Pro Val Asp Ile Glu Gln Glu Met Gln Arg Ser Tyr Ile Asp Tyr Ala
            20                  25                  30
Met Ser Val Ile Val Gly Arg Ala Leu Pro Glu Val Arg Asp Gly Leu
        35                  40                  45
Lys Pro Val His Arg Arg Val Leu Tyr Ala Met Phe Asp Ser Gly Phe
    50                  55                  60
Arg Pro Asp Arg Ser His Ala Lys Ser Ala Arg Ser Val Ala Glu Thr
65                  70                  75                  80
Met Gly Asn Tyr His Pro His Gly Asp Ala Ser Ile Tyr Asp Ser Leu
                85                  90                  95
Val Arg Met Ala Gln Pro Trp Ser Leu Arg Tyr Pro Leu Val Asp Gly
            100                 105                 110
Gln Gly Asn Phe Gly Ser Pro Gly Asn Asp Pro Pro Ala Ala Met Arg
        115                 120                 125
Tyr Thr Glu Ala Arg Leu Thr Pro Leu Ala Met Glu Met Leu Arg Glu
    130                 135                 140
Ile Asp Glu Glu Thr Val Asp Phe Ile Pro Asn Tyr Asp Gly Arg Val
145                 150                 155                 160
Gln Glu Pro Thr Val Leu Pro Ser Arg Phe Pro Asn Leu Leu Ala Asn

                165                 170                 175
Gly Ser Gly Gly Ile Ala Val Gly Met Ala Thr Asn Ile Pro Pro His
            180                 185                 190
Asn Leu Arg Glu Leu Ala Asp Ala Val Phe Trp Ala Leu Glu Asn His
        195                 200                 205
Asp Ala Asp Glu Glu Glu Thr Leu Ala Ala Val Met Gly Arg Val Lys
    210                 215                 220
Gly Pro Asp Phe Pro Thr Ala Gly Leu Ile Val Gly Ser Gln Gly Thr
225                 230                 235                 240
Ala Asp Ala Tyr Lys Thr Gly Arg Gly Ser Ile Arg Met Arg Gly Val
                245                 250                 255
Val Glu Val Glu Glu Asp Ser Arg Gly Arg Thr Ser Leu Val Ile Thr
            260                 265                 270
Glu Leu Pro Tyr Gln Val Asn His Asp Asn Phe Ile Thr Ser Ile Ala
        275                 280                 285
Glu Gln Val Arg Asp Gly Lys Leu Ala Gly Ile Ser Asn Ile Glu Asp
    290                 295                 300
Gln Ser Ser Asp Arg Val Gly Leu Arg Ile Val Ile Glu Ile Lys Arg
305                 310                 315                 320
Asp Ala Val Ala Lys Val Val Ile Asn Asn Leu Tyr Lys His Thr Gln
                325                 330                 335
Leu Gln Thr Ser Phe Gly Ala Asn Met Leu Ala Ile Val Asp Gly Val
            340                 345                 350
Pro Arg Thr Leu Arg Leu Asp Gln Leu Ile Arg Tyr Tyr Val Asp His
        355                 360                 365
Gln Leu Asp Val Ile Val Arg Arg Thr Thr Tyr Arg Leu Arg Lys Ala
    370                 375                 380
Asn Glu Arg Ala His Ile Leu Arg Gly Leu Val Lys Ala Leu Asp Ala
385                 390                 395                 400
Leu Asp Glu Val Ile Ala Leu Ile Arg Ala Ser Glu Thr Val Asp Ile
                405                 410                 415
Ala Arg Ala Gly Leu Ile Glu Leu Leu Asp Ile Asp Glu Ile Gln Ala
            420                 425                 430
Gln Ala Ile Leu Asp Met Gln Leu Arg Arg Leu Ala Ala Leu Glu Arg
        435                 440                 445
Gln Arg Ile Ile Asp Asp Leu Ala Lys Ile Glu Ala Glu Ile Ala Asp
    450                 455                 460
Leu Glu Asp Ile Leu Ala Lys Pro Glu Arg Gln Arg Gly Ile Val Arg
465                 470                 475                 480
Asp Glu Leu Ala Glu Ile Val Asp Arg His Gly Asp Asp Arg Arg Thr
                485                 490                 495

Arg Ile Ile Ala Ala Asp Gly Asp Val Ser Asp Glu Asp Leu Ile Ala
            500                 505                 510
Arg Glu Asp Val Val Val Thr Ile Thr Glu Thr Gly Tyr Ala Lys Arg
        515                 520                 525
Thr Lys Thr Asp Leu Tyr Arg Ser Gln Lys Arg Gly Gly Lys Gly Val
    530                 535                 540
Gln Gly Ala Gly Leu Lys Gln Asp Asp Ile Val Ala His Phe Phe Val
545                 550                 555                 560
Cys Ser Thr His Asp Leu Ile Leu Phe Phe Thr Thr Gln Gly Arg Val
                565                 570                 575
Tyr Arg Ala Lys Ala Tyr Asp Leu Pro Glu Ala Ser Arg Thr Ala Arg
            580                 585                 590
Gly Gln His Val Ala Asn Leu Leu Ala Phe Gln Pro Glu Glu Arg Ile
        595                 600                 605
Ala Gln Val Ile Gln Ile Arg Gly Tyr Thr Asp Ala Pro Tyr Leu Val
    610                 615                 620
Leu Ala Thr Arg Asn Gly Leu Val Lys Lys Ser Lys Leu Thr Asp Phe
625                 630                 635                 640
Asp Ser Asn Arg Ser Gly Gly Ile Val Ala Val Asn Leu Arg Asp Asn
                645                 650                 655
Asp Glu Leu Val Gly Ala Val Leu Cys Ser Ala Gly Asp Asp Leu Leu
            660                 665                 670
Leu Val Ser Ala Asn Gly Gln Ser Ile Arg Phe Ser Ala Thr Asp Glu
        675                 680                 685
Ala Leu Arg Pro Met Gly Arg Ala Thr Ser Gly Val Gln Gly Met Arg
    690                 695                 700
Phe Asn Ile Asp Asp Arg Leu Val Ser Leu Asn Val Val Arg Glu Gly
705                 710                 715                 720
Thr Tyr Leu Leu Val Ala Thr Ser Gly Gly Tyr Ala Lys Arg Thr Ala
                725                 730                 735
Ile Glu Glu Tyr Pro Val Gln Gly Arg Gly Gly Lys Gly Val Leu Thr
            740                 745                 750
Val Met Tyr Asp Arg Arg Arg Gly Arg Leu Val Gly Ala Leu Ile Val
        755                 760                 765
Asp Asp Asp Ser Glu Leu Tyr Ala Val Thr Ser Gly Gly Gly Val Ile
    770                 775                 780
Arg Thr Ala Ala Arg Gln Val Arg Lys Ala Gly Arg Gln Thr Lys Gly
785                 790                 795                 800
Val Arg Leu Met Asn Leu Gly Glu Gly Asp Thr Leu Leu Ala Ile Ala
                805                 810                 815
Arg Asn Ala Glu Glu Ser Gly Asp Asp Asn Ala Val Asp Ala Asn Gly

820                    825                    830
Ala Asp Gln Thr Gly Asn
        835

<210> 5
<211> 23
<212> DNA
<213> Mycobacterium tuberculosis

<220>
<221> CDS
<222> (2)..(49)

<400> 5
agcgcagcta catcgactat gcg                                              23

<210> 6
<211> 23
<212> DNA
<213> Mycobacterium tuberculosis

<220>
<221> CDS
<222> (2)..(49)

<400> 6
cttcggtgta cctcatcgcc gcc                                              23

## Description

### BACKGROUND OF THE INVENTION

### Field of the Invention

The present invention relates to a method and kit for determining quinolon resistance of srtains of tubercle bacilli (*Mycobacterium tuberculosis*), more specifically, a method and kit for determining quinolon resistance by detecting a novel mutation in the gyrase A gene, which imparts the quinolon resistance phenotype to tubercle bacilli.

### Description of the Related Art

Drug resistance phenotype acquisition mechanisms of bacteria include (1) such change of the cell wall structure that permeation of a drug into a cell should be blocked, (2) acquisition of a gene coding for an enzyme that decomposes or inactivates a drug, (3) change of a compound in a cell as a target of drug and so forth. Acquisition of these phenotypes are acheived by transmission among bacteria via transposable genetic elements such as plasmid and transposon, change of chromosomes or the like.

Acquisition of resistance phenotype to drugs (hereinafter, referred to as "antituberculous drug") by *Mycobacterium tuberculosis* (hereinafter, also referred to as "tubercle bacilli") is attributable to a spontaneous chromosomal mutation (A. Telenti, Tuberculosis, 1997, 18 (1): 55-64). For example, the mechanism of resistance of tubercle bacilli to rifampicin, which is an antituberculous drug, is resulted from nucleotide substitution occurring in the gene encoding the RNA polymerase β subunit protein, which is a target substance of rifampicin (A Telenti et al., Lancet, 1993, 341: 647-650). Furthermore, nucleotide substitution in the *rpsL* and *rrs* genes or the like induces streptomycin resistance (Y. Suzuki, et al., J. Applied. Microbiol., 1997, 83: 634-640). Thus, mechanisms for acquisition of resistance phenotype by a mutation in a gene involved in each target molecule exist for some antituberculous drugs.

Accordingly, most of drug resistance phenotype acquisition mechanisms of tubercle bacilli are explained based on spontaneous mutations.

In *Escherlchia coli*, bacteria belonging to the genus *Staphylococcus* and so forth, quinolon resistance is acquired by introduction of a nucleotide mutation into a gene encoding the DNA gyrase activity, topoisomerase IV activity or a membrane integral protein involved in uptake and excretion of a substance into and from a cell (Cambau E., et al., Res. Microbiol., 1996, 147: 52-59; Ferrero L., et al., Antimicrob. Agents Chemother., 1995, 39: 1554-1558) or the like. One of the target compounds of quinolon is gyrase A, and a mutation in the gyrase A gene greatly contributes to resistance to clinically important quinolon molecular species (ciprofloxacin, ofloxacin etc., Cambau E., et al., J. Infect. Dis., 1994, 170: 479-483).

There have also been reported several mutations in the gyrase A gene involved in acquisition of quinolon resistance phenotype in tubercle bacilli (Antimicrob. Agents Chemother., 1996, 40 (8) , 1768-1774; J. Infect. Dis., 1996, 174, 1127-1130; Eur. J. Clin. Microbiol. Infect. Dis., 1997, 16, 395-398; J. Infect. Dis., 2000, 182, 517-525).

### Summary of the Invention

An object of the present invention is to provide a method and kit for determining quinolon resistance of tubercle bacilli by identifying a novel mutation in the gyrase A gene involved in acquisition of quinolon resistance phenotype in tubercle bacilli and utilizing the mutation.

The inventors of the present invention assiduously studied in order to achieve the aforementioned object. As a result, they found a novel mutation imparting a quinolon resistance phenotype in the gyrase A gene sequence of tubercle bacilli and accomplished the present invention.

That is, the present invention provides the followings.
(1) A method for determining quinolon resistance of a *Mycobacterium tuberculosis* strain by detecting a mutation in a gyrase A gene of the bacterial strain, wherein the mutation is substitution of another amino acid residue for an amino acid residue corresponding to the 89th aspartic acid residue in an amino acid sequence encoded by the gyrase A gene.
(2) The method according to (1), wherein the other amino acid residue is an asparagine residue.
(3) The method according to (1) or (2), wherein the amino acid sequence encoded by the gyrase A gene is the amino acid sequence of SEQ ID NO: 4 or an amino acid sequence of SEQ ID NO: 4 including substitution, deletion or insertion of one or several amino acid residues.
(4) The method according to any one of (1) to (3), wherein the mutation is substitution of T for G that is the first nucleotide of a codon coding for an aspartic acid residue.
(5) The method according to (4), wherein the mutation occurs at a position corresponding to the 530th nucleotide in the nucleotide sequence of SEQ ID NO: 3.
(6) The method according to any one of (1) to (5), further comprising detecting a substitution of A for a base corresponding to G at the 545th position in the nucleotide sequence of SEQ ID NO: 3.
(7) A kit for determining quinolon resistance of a *Mycobacterlum tuberculosis* strain by detecting a mutation in a gyrase A gene of the bacterial strain, comprising an oligonucleotide for detecting a substitution of another amino acid residue for an amino acid residue corresponding to the 89th aspartic acid residue in an amino acid sequence encoded by the gyrase A gene.

A novel mutation in the gyrase A gene involved in quinolon resistance phenotype acquisition by tubercle bacilli was identified by the present invention. By utilizing this mutation, a novel method and kit for determining quinolon resistance of tubercle bacilli are provided.

### Brief Explanation of the Drawings

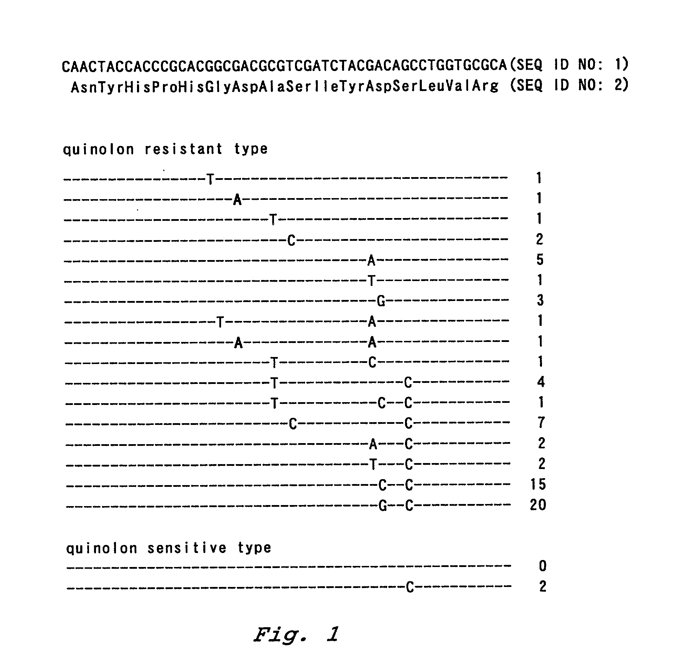

Fig. 1 shows alignment of nucleotide sequences of *gyrA* genes from strains of tubercle bacilli resistant to quinolon.

### Detailed Description of the Preferred Embodiments

Hereafter, the present invention will be explained in detail.

The present invention provides a method for determining quinolon resistance of a tubercle bacillus strain by detecting a mutation in the gyrase A gene (hereinafter, referred to as "*gyrA*") of the bacterial strain. Within the reported nucleotide sequence of the *gyrA* gene of wild tubercle bacillus strain (GenBank locus MSGGYRAB, accession L27512.1 GI: 1107467), the sequence of nucleotide numbers 2041-4923 is shown as SEQ ID NO: 3. Further, an amino acid sequence encoded by the nucleotide sequence is shown as SEQ ID NO: 4. The amino acid sequence is shown in both of SEQ ID NOS: 3 and 4.

The method of the present invention is characterized in that the mutation in the aforementioned *gyrA* gene is a substitution of another amino acid residue for an amino acid residue corresponding to the 89th aspartic acid residue in the amino acid sequence encoded by the *gyrA* gene. Fig. 1 shows comparison of nucleotide sequences of a region around the mutation point. Further, a nucleotide sequence of the aforementioned region in the *gyrA* gene of a wild strain is shown as SEQ ID NO: 1. The amino acid sequence encoded by this sequence is shown as SEQ ID NO: 2. SEQ ID NO: 1 corresponds to the nucleotide numbers 511-560 in the nucleotide sequence including the *gyrA* gene shown as SEQ ID NO: 3. Further, the amino acid sequence of SEQ ID NO: 2 corresponds to the amino acid numbers 83-98 in the amino acid sequence encoded by the *gyrA* gene and shown as SEQ ID NO: 4.

As the aforementioned other amino acid residue, an asparagine residue can be mentioned. As a mutation in the *gyrA* gene that induces a mutation for substituting an asparagine residue for the aspartic acid residue, substitution of T for G that is the first nucleotide of a codon coding for an aspartic acid residue can be mentioned. A specific example of this mutation is a mutation occurring at a position corresponding to the 530th nucleotide in the nucleotide sequence of the *gyrA* gene shown as SEQ ID NO: 3.

The nucleotide or amino acid numbers in the nucleotide sequence or the amino acid sequence shown as SEQ ID NO: 3 or 4 may be changed due to a mutation in the *gyrA* gene occurring at a position that is not within a codon corresponding to the 89th aspartic acid residue. A gyrase A having such a mutation has an amino acid sequence including substitution, deletion or insertion of one or several amino acids in the amino acid sequence shown as SEQ ID NO: 4. The present invention can also be applied to a gene coding for the gyrase A including such a mutation so long as the mutation is involved in quinolon resistance. The expressions "amino acid residue corresponding to the 89th aspartic acid residue" and "position corresponding to the 530th nucleotide" refer to an amino acid residue or a nucleotide residue corresponding to such a position in the sequence shown as SEQ ID NO: 3 or 4 when the nucleotide numbers or amino acid numbers are changed as described above. For example, when the first amino acid residue of SEQ ID NO: 4 is deleted, an amino acid residue corresponding to the 89th aspartic acid residue means an aspartic acid residue that is the 88th amino acid residue from the N-terminus.

In the present invention, known mutations in the *gyrA* gene involved in quinolon resistance and/or mutations yet to be found in future may be detected in addition to the substitution of an amino acid residue corresponding to the 89th aspartic acid residue. As such mutations, there can be mentioned substitution of A for a base corresponding to G at the 545th position in SEQ ID NO: 3.

Mutations in the *gyrA* gene of tubercle bacillus can be detected in the same manner as in usual mutation detecting methods so long as a single nucleotide polymorphism can be detected. For example, there can be mentioned a method for detecting a mutation by directly determining the nucleotide sequence of the *gyrA* gene. Further, examples of a method for detecting a mutation utilizing PCR include methods such as the template-directed dye-terminator incorporation method (Chen, X. et al., Proc. Natl. Acad. Sci. USA, 20, 10756-10761 (1997)), the dye-labeled oligonucleotide ligation method (Chen, X. et al., Genome Res., 5, 549-556 (1998)), the molecular beacon method (Tyagi, S. et al., Nat. Biotechnol., 1, 49-53 (1998)), the dynamic allele-specific hybridization method (Howellm, W.M. et al., Nat. Biotechnol., 1, 87-88 (1998)) and so forth.

Further, as methods that do not utilize PCR, there can be mentioned the padlock probe method developed by Mats Nilsson et al. in 1994 (Nilsson, M. et al., Science, 5181, 2085-2088 (1994)) and modified by Paul M. Lizardi et al. (Lizardi, P. et al., Nat. Genet., 3, 225-232 (1998)), the invader assay method (WO94/29482) developed by Victor Lyamichev and so forth.

Furthermore, as other methods for detecting a single nucleotide polymorphism, the Luminex method utilizing flow cytometry and methods utilizing matrix-assisted laser desorption ionization mass spectrometry (MALDI-TOFMS) are also known and can be used for the present invention.

Further, microarray methods (WO95/11995, USP 5,837,832), which are widely used in recent years, such as the single base sequencing on oligomicroarray method (Drmanac, R. et al., Genomics, 2, 114-128 (1989)) can also be used. The microarray methods are techniques in which a large number of capture nucleic acids are immobilized on a solid phase, a labeled target nucleic acid is hybridized with the capture nucleic acids to be captured, and the presence or absence or existence ratio of the target nucleic acid is analyzed exhaustively.

As an example of methods preferably used in the present invention, a method of detecting a target mutation in the *gyrA* gene by using an oligonucleotide immobilized on a substrate will be shown below.

As the oligonucleotide immobilized on the substrate, used is an oligonucleotide having a nucleotide sequence of a region including the position of the aforementioned mutation in the *gyrA* gene (hereinafter, referred to as "capture oligo").

When the capture oligo is designed, it is usually preferred that a position corresponding to the aforementioned mutation included in the capture oligo should exist in the center portion of the capture oligo. When the capture oligo is too short, detection of the hybridization becomes difficult. When it is too long, inhibition of the hybridization due to a sequence unique to that type does not occur. Therefore, a length in the range of 10 to 24 nucleotides is preferred. Further, when there is a secondary structural failure that negatively affects the hybridization in a process of hybrid formation of the capture oligo and a target described later, a spacer or a nucleotide that does not form a hydrogen bond with any nucleotide can be introduced into the oligonucleotide sequence to avoid the aforementioned failure.

Although DNA is usually used as the capture oligo, a peptide nucleic acid (PNA) may also be used. Since a hybrid formed by a peptide nucleic acid with a nucleic acid derived from a test human genome has a higher Tm (melting temperature) in comparison with that obtained by using an oligonucleotide, it is expected that a stable hybridization signal can be obtained. The peptide nucleic acid can be readily synthesized by using a usual peptide synthesizer.

Synthesis of the oligonucleotide, preparation of chromosomal DNA, hybridization and PCR can be performed according to usual methods well known to those skilled in the art (refer to Maniatis, T. et al., "Molecular Cloning A Laboratory Manual, Second Edition", Cold Spring Harbor Laboratory Press (1989)). Further, the oligonucleotide can be synthesized by using a commercially available DNA synthesizer.

Material of the substrate on which the oligonucleotide is immobilized is not particularly limited so long as the oligonucleotide can be stably immobilized thereon. However, for example, glass, synthetic resins such as polycarbonate and plastic can be mentioned. Although shape of the substrate is not also particularly limited, a plate-like or film-like substrate can be mentioned. The substrate preferably has a uniform and planar surface.

The oligonucleotide can be immobilized on the substrate by using a method used in a usual hybridization method such as physical adsorption, electrical coupling and formation of a molecular covalent bond. For example, there can be mentioned a method using a substrate of which surface is coated with a compound having a carbodiimide group or an isocyanate group (Japanese Patent Laid-open Publication (Kokai) No. 08-023975). The substrate coated with a compound having a carbodiimide group or an isocyanate group on the surface thereof can be prepared by coating the substrate surface with a macromolecular compound having a carbodiimide group or an isocyanate group. The oligonucleotides can be immobilized with a covalent bond on the substrate by irradiating with a ultraviolet ray. Further, as a linker for bonding the carbodiimide group or isocyanate group and the oligonucleotide, a compound having an amino group or an imino group having high reactivity with the carbodiimide group or isocyanate group is used. In the case of imino group, the compound can be bonded with a carbodiimide group or an isocyanate group by polymerizing thymine to either one of the ends of capture oligo.

The oligonucleotide can be immobilized on the substrate by, for example, spotting an oligonucleotide solution on the substrate by using a spotting machine. Usually, the oligonucleotide solution is preferably spotted in a substantially circular shape.

Further, multiple kinds of oligonucleotide are each usually spotted at multiple positions on a single substrate, and these spots are preferably arranged in a grid pattern. When the spot size is 1000 µm in diameter, the total number of spots is preferably 1600 spots/cm² or less and 40 x 40 or less of spots are preferably provided when they are spotted in a square pattern. Further, when the spot size is 10 µm in diameter, the total number is preferably 400 spots/cm² or less, and 20 x 20 or less of spots are preferably provided when they are spotted in a square pattern. Further, when the vertical and horizontal sizes of the pattern are different, the numbers of the spots along the vertical and horizontal directions may be adjusted depending on shape of the pattern.

The presence or absence of a specific mutation in the *gyrA* gene can be determined by hybridizing a nucleic acid fragment probe including a region corresponding to the position of the aforementioned mutation in the *gyrA* gene of chromosomal DNA of tubercle bacillus (referred to as "nucleic acid probe") with a wild type capture oligo and a mutant capture oligo and examining with which type of capture oligo the probe hybridized. That is, to which drug the strain has resistance can be detected depending on the hybridization position on the substrate.

The nucleic acid can be prepared from tubercle bacilli in the same manner as in a usual method for preparing nucleic acids from bacteria. For example, DNA can be prepared according to the method described in Maniatis, T. et al., "Molecular Cloning A Laboratory Manual, Second Edition", Cold Spring Harbor Laboratory Press (1989).

The nucleic acid probe can be prepared by amplifying a nucleic acid by using primers designed based on the nucleotide sequence of the capture oligo. Although DNA is usually used as the nucleic acid probe, RNA may also be used. As the method for amplifying a nucleic acid, there can be mentioned, for example, a method of amplifying a nucleic acid as DNA by polymerase chain reaction (PCR) and a method of amplifying a nucleic acid as RNA by the in vitro transcription method.

The primers used in PCR are designed so that the nucleic acid probe should include a specific mutation site in the *gyrA* gene and sequences on both sides thereof. As such primers, there can be mentioned oligonucleotides having the nucleotide sequences shown as SEQ ID NOS: 5 and 6.

If a primer is labeled beforehand, a labeled nucleic acid probe can be obtained. The nucleic acid probe may also be labeled during or after the nucleic acid amplification reaction. As a labeling substance, labeling substances similar to those used for a probe employed in usual hybridization such as a fluorescence substance or hapten can be used. Specific examples of the fluorescence substance include fluorescein (FITC), Rhodamine, phycoerythrin (PE), Texas Red, cyanine fluorescent dyes and so forth. Specific examples of the hapten include biotin, digoxigenin (Dig), dinitrophenyl (DNP) and so forth.

The primers for preparing a nucleic acid probe can be included in a kit for determining quinolon resistance of tubercle bacilli together with a substrate on which the oligonucleotide is immobilized.

The hybridization can be performed in the same manner as in usual nucleic acid hybridization. A specific method will be exemplified below.

A nucleic acid probe is added to a mixed solution containing a salt solution such as SSC (standard saline citrate), a blocking solution containing sodium dodecylsulfate (SDS), bovine serum albumin (BSA) or the like and additives for promoting a hybridization reaction. When the target is a double strand, denaturation is performed by heating or the like. A nucleic acid probe solution is added onto a substrate in an amount of several µL and heated for several hours (usually 37-70°C) to form a hybrid between an oligonucleotide immobilized on the substrate and the nucleic acid probe.

A solution of 5 x SSC or 3 M tetramethylammonium chloride is added onto the substrate and heated (usually 37-50°C) to remove oligonucleotides that form a nonspecific hybrid or do not form specific hybrid from the substrate so that only specific hybrids should be selectively left on the substrate.

The hybrids are detected by using the fluorescence substance or hapten introduced into the nucleic acid probe. When a hapten is used, a solution containing a conjugate of a protein that recognizes the hapten or protein that bonds to the hapten and alkaline phosphatase, horseradish peroxidase or the like (enzyme conjugate) is added onto the substrate and allowed to react at room temperature for several dozens of minutes. A nonspecific adsorption reaction of the enzyme conjugate and the substrate can be prevented by completely coating regions on the substrate with a protein such as BSA except for the regions in which the oligonucleotides are immobilized before a bonding reaction of the hapten and the enzyme conjugate is performed. This treatment can be performed by, after the oligonucleotides are immobilized, adding a solution of a protein such as BSA onto the substrate and leaving it at room temperature for several dozens of minutes. After the bonding reaction of the enzyme conjugate and the hapten of the nucleic acid probe is completed, only enzyme conjugates that bond to the hapten in the nucleic acid probe are left on the substrate by washing the substrate with an appropriate buffer containing a surfactant to remove enzyme conjugates that did not bond to the hapten.

In order to visualize the hybrids, a compound that becomes insoluble only when only a bonding product of the hapten and the enzyme conjugate exists is added. The production of the insoluble compound is amplified by the enzymatic reaction, and thus the hybrids are visualized. As the compound used for this purpose, nitroblue tetrazolium chloride (NBT) and 5-bromo-4-chloro-3-indolyl phosphate, p-toluidine salt (BCIP) are used when the enzyme in the enzyme conjugate is alkaline phosphatase. When the enzyme is horseradish peroxidase, 3,3',5,5'-tetramethylbenzidine (TMB) or the like can be used.

Quinolon resistance of tubercle bacillius is determined based on the obtained results of the hybridization by examining pigmentation or fluorescent color development at positions at which the capture oligos are immobilized.

The kit of the present invention is a kit for determining quinolon resistance of a tubercle bacillus strain by detecting a mutation in the *gyrA* gene of the bacterial strain, which includes an oligonucleotide for detecting a substitution of another amino acid residue for an amino acid residue corresponding to the 89th aspartic acid residue in the amino acid sequence encoded by the gyrase A gene. As the oligonucleotide, a capture oligo or a substrate on which the capture oligo is immobilized can be mentioned. Further, the kit of the present invention may include primers for preparing a nucleic acid probe, labeled nucleic acid probe, buffer, reagents for hybridization such as enzyme conjugate that recognizes hapten and so forth.

As quinolon to which the present invention can be applied, there can be mentioned levofloxacin, ciprofloxacin, ofloxacin, gatifloxacin and so forth.

### Examples

Hereafter, the present invention will be explained more specifically with reference to the following example.

### <1> Isolation of quinolon-resistant strain of Mycobacterium tuberculosis

Sputum of a tuberculosis patient was applied on Ogawa medium and cultured in an incubator at 37°C for 4 weeks. Appeared colonies were inoculated again on Ogawa medium containing 2.5 µg/mL of levofloxacin and cultured in an incubator at 37°C for 4 weeks. The strains that formed colonies were isolated as quinolon-resistant strains.

### <2> Determination of nucleotide sequence of gyrA gene of quinolon-resistant strain

Genomic DNA was prepared from each of the quinolon-resistant strains isolated as described above by the method of Kent et al. (Kent, P.T., et al., Mycobacteriology, A Guide for the Level III Laboratory, p.31, U.S. Department of Health and Human Service, Public Health Service, Center for Disease Control, 1985). PCR was performed for the obtained genome by using primers having the nucleotide sequences shown as SEQ ID NOS: 5 and 6.

rTaq (Takara Shuzo) was used for PCR. PCR was performed by a reaction at 98°C for 1 minute, 35 cycles of reactions at 98°C for 5 seconds, at 55°C for 10 seconds and at 72°C for 20 seconds, and a reaction at 72°C for 1 minute. The amplification product was separated by 1% agarose gel electrophoresis, and a gel containing a 0.4 kbp band was excised. This gel piece was placed into a 1.5-mL micro tube and frozen at -135°C for 15 minutes. Then, the gel was centrifuged at 15000 rpm for 10 minutes to obtain a supernatant. Resulting DNA solution was used as a template together with a primer shown as SEQ ID NO: 5 and Big Dye Terminator kit manufactured by ABI to perform a sequencing reaction. After completion of the reaction, the sequence was analyzed by using a genetic analyzer manufactured by ABI.

Fig. 1 shows alignment of the obtained sequences. In Fig. 1, the number shown at the right end of each sequence represents the number of sequences that had the same mutation among the determined sequences. Among these mutations, substitution of A for G at the 530th position in the nucleotide sequence shown as SEQ ID NO: 3 is a novel mutation. Further, substitution of A for G at the 530th position and substitution of A for G at the 545th position constitute a novel combination of mutations. Other mutations were the same as the mutations already reported (Antimicrob. Agents Chemother., 1996, 40 (8), 1768-1774; J. Infect. Dis., 1996, 174, 1127-1130; Eur. J. Clin. Microbiol. Infect. Dis., 1997, 16, 395-398; J. Infect. Dis., 2000, 182, 517-525).

## Claims

1. A method for determining quinolon resistance of a *Mycobacterium tuberculosis* strain comprising detecting a mutation in a gyrase A gene of the bacterial strain, wherein the mutation is substitution of another amino acid residue for an amino acid residue corresponding to the 89th aspartic acid residue in the amino acid sequence encoded by the gyrase A gene.

2. The method according to claim 1, wherein the other amino acid residue is an asparagine residue.

3. The method according to claim 1 or 2, wherein the amino acid sequence encoded by the gyrase A gene is the amino acid sequence of SEQ ID NO: 4 or an amino acid sequence of SEQ ID NO: 4 including substitution, deletion or insertion of one or several amino acid residues.

4. The method according to any one of claims 1 to 3, wherein the mutation is substitution of T for G that is the first nucleotide of a codon coding for an aspartic acid residue.

5. The method according to claim 4, wherein the mutation occurs at a position corresponding to the 530th nucleotide in the nucleotide sequence of SEQ ID NO: 3.

6. The method according to any one of claims 1 to 5, further comprising detecting a substitution of A for a base corresponding to G at the 545th position in the nucleotide sequence of SEQ ID NO: 3.

7. A kit for determining quinolon resistance of a *Mycobacterium tuberculosis* strain by detecting a mutation in a gyrase A gene of the bacterial strain, comprising an oligonucleotide for detecting a substitution of another amino acid residue for an amino acid residue corresponding to the 89th aspartic acid residue in an amino acid sequence encoded by the gyrase A gene.
